# FASCICULE DE BREVET EUROPEEN

(11) **EP 1 102 586 B1**
(45) Date de publication et mention de la délivrance du brevet: **03.11.2004**
(21) Numéro de dépôt: 99936702.2
(22) Date de dépôt: 06.08.1999
(51) Int. Cl.: A61K 31/365, A61K 35/78

(54) **UTILISATION D'EXTRAITS DE GINKGO BILOBA POUR PREPARER UN MEDICAMENT DESTINE A TRAITER LA SCLEROSE LATERALE AMYOTROPHIQUE**
VERWENDUNG VON GINGKO BILOBA EXTRAKTEN ZUR HERSTELLUNG EINES ARZNEIMITTELS ZUR BEHANDLUNG DER AMYOTROPHEN LATERALSKLEROSE
USE OF GINKGO BILOBA EXTRACTS FOR PREPARING A MEDICINE FOR TREATING AMYOTROPHIC LATERAL SCLEROSIS

(30) Priorité: 07.08.1998 FR 9810187
(43) Date de publication de la demande: 30.05.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHRISTEN, Yves, F-75116 Paris (FR)
(74) Mandataire: Bourgouin, André
(86) Numéro de dépôt international: PCT/FR1999/001948
(87) Numéro de publication internationale: WO 2000/007592

(56) Documents cités:
- EP-A- 0 143 977
- EP-A- 0 436 129
- WO-A-97/15304
- DE-A- 4 328 374
- BRUNO C ET AL: "Regeneration of motor nerves in bilobalide-treated rats." PLANTA MEDICA, (1993 AUG) 59 (4) 302-7. JOURNAL CODE: P9F. ISSN: 0032-0943., XP002100522 GERMANY: Germany, Federal Republic of
- BRAILOWSKY S ET AL: "Motor function in young and aged hemiplegic rats: effects of a Ginkgo biloba extract." NEUROBIOLOGY OF AGING, (1997 MAR-APR) 18 (2) 219-27. JOURNAL CODE: NX5. ISSN: 0197-4580., XP002100523 United States
- SEEBURGER J L ET AL: "EXPERIMENTAL RATIONALE FOR THE THERAPAUTIC USE OF NEUROTROPHINS IN AMYOTROPHIC LATERAL SCLEROSIS" EXPERIMENTAL NEUROLOGY, vol. 124, no. 1, novembre 1993 (1993-11), pages 64-72, XP002008240
- LINDVALL O ET AL: "CLINICAL APPLICATION OF CELL TRANSPLANTATION AND NEUROTROPHIC FACTORS IN CNS DISORDERS" CURRENT OPINION IN NEUROBIOLOGY, vol. 4, no. 5, 1994, pages 752-757, XP002008239
- GUINOT, PHILIPPE ET AL: "Effects of the PAF antagonists, ginkgolides (BN 52063, BN 52021), in various clinical indications" J. LIPID MEDIATORS CELL SIGNALLING (1994), 10(1-2), 141-6 CODEN: JLMSEO;ISSN: 0929-7855, XP002100524

## Description

L'invention concerne l'utilisation d'extraits de Ginkgo biloba pour préparer un médicament destiné à traiter la sclérose latérale amyotrophique (SLA).

Il est déjà connu que les extraits de Ginkgo biloba présentent une activité dans le domaine cardio-vasculaire (notamment la réduction de l'adhésion des plaquettes), dans le domaine nerveux central (notamment une activité neuroprotective) ou dans le système neuro-sensoriel (notamment une protection rétinienne) ; cf. par exemple DeFeudis et coll., Ginkgo Biloba Extract (EGb 761), Pharmaceutical Activities and Clinical applications (Elsevier, Paris, 1991). Leur préparation a fait l'objet d'un certain nombre de brevets, parmi lesquels on peut citer les brevets européens EP 431 535 et EP 431 536, et le brevet américain US 5,389,370.

Certains produits peuvent être utilisés dans le traitement de la SLA. On peut notamment citer le riluzole, la gabapentine, la 1-(2-napht-2-yléthyl)-4-(3-trifluorométhylphényl)-1,2,3,6-tétrahydropyridine ou la vitamine E (cf. Gurney M.E. et coll., *Ann. Neurol.,* **39** (1996), 147-157 ; demande de brevet PCT WO 97/15304).

Or la demanderesse vient de trouver que certains extraits de Ginkgo biloba possèdent en . outre de nouvelles propriétés pharmacologiques intéressantes, à savoir retarder et atténuer les symptomes de la SLA. Elle a en particulier pu constater les effets bénéfiques de ces extraits sur des souris génétiquement modifiées atteintes de SLA.

L'invention a donc pour objet l'utilisation de ces extraits pour préparer un médicament destiné à traiter la SLA.

Par extrait de Ginkgo biloba, on entend au moins un des composés individuels qui puisse être obtenu par extraction de l'arbre de *Ginkgo biloba L*., et notamment un composé flavonoïdique ou un terpène tel un ginkgolide ou un bilobalide, ou encore un mélange de ces derniers. De préférence, l'extrait utilisé sera tel qu'il contienne une quantité efficace de ginkgolides. Pour les utilisations selon l'invention, on pourra par exemple choisir un extrait de type EGb 761 ou CP 401.

Par ginkgolide, on entend tous les ginkgolides naturels obtenus à partir de l'arbre de Ginkgo biloba, ainsi que les ginkgolides synthétiques et leurs dérivés (résultant par exemple d'une réaction d'acétylation ou d'alkoxylation) et sels pharmaceutiquement actifs. Les ginkgolides utilisés pourront par exemple être le ginkgolide A, le ginkgolide B, le ginkgolide C, le ginkgolide J ou le ginkgolide M (structures données dans le schéma ci-dessous ; ces composés peuvent être isolés à partir d'extraits de feuilles de *Ginkgo biloba* - voir *GINKGOLIDES, Chemistry, Biology, Pharmacology and Clinical Perspectives,* Edité par P. Braquet, J.R. Prous Science Publishers, notamment Volumes 1 (1988) et 2 (1989)). On pourra également utiliser des dérivés glycosylés de ginkgolides ou de dérivés alkoxylés ou acétylés de ginkgolides (cf. Weber, M. et Vasella, A., *Helv. Chim. Acta*, **80** (1997), 2352-2367). Par dérivé alkoxylé de ginkgolide, on entend un dérivé de ginkgolide comportant au moins un groupe alkoxy, linéaire ou ramifié, à la place d'un groupe hydroxy (ces composés sont décrits dans la demande de brevet français FR 88.14392). De la même façon, par dérivé acétylé de ginkgolide, on entend un dérivé de ginkgolide comportant au moins un groupe acétate à la place d'un groupe hydroxy.

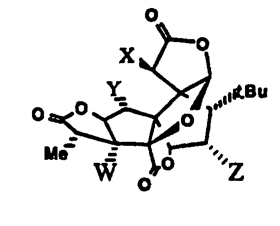

| *Structure des ginkgolides A, B C, J et M* | | | | |
|---|---|---|---|---|
| Ginkgolide | W | X | Y | Z |
| A | OH | OH | H | H |
| B | OH | OH | OH | H |
| C | OH | OH | OH | OH |
| J | OH | OH | H | OH |
| M | H | OH | OH | OH |

Par extrait de type EGb 761, on entend un extrait de composition sensiblement identique à celle de l'extrait standardisé EGb 761 tel qu'il a été défini notamment dans l'article suivant : K. Drieu, La presse médicale, **31**, 25 septembre 1986, supplément consacré à l'extrait de Ginkgo biloba (EGb 761), 1455-1457 ; ou dans les brevets européens EP 431 535 et EP 431 536 ; par extrait de type EGb 761, on entend donc notamment des extraits de Ginkgo biloba comprenant de 20 à 30 % de flavoneglycosides, de 2,5 à 4,5 % de ginkgolides A, B, C et J, de 2 à 4 % de bilobalide, moins de 10 % de proanthocyanidines et moins de 10 ppm, et de préférence moins de 5 ppm, de composés de type alkylphénols, et en particulier les extraits de Ginkgo biloba comprenant environ 24 % de flavoneglycosides, 3,1 % de ginkgolides A, B, C et J, 2,9 % de bilobalide, 6,5 % de proanthocyanidines et moins de 1 ppm de composés de type alkylphénols. Par extrait de type CP 401, on entend des extraits tels que ceux qui sont présentés dans le brevet US 5,389,370, notamment les extraits de Ginkgo biloba contenant de 5,5 à 8 % de ginkgolides A, B, C et J, de 40 à 60 % de flavoneglycosides et de 5 à 7 % de bilobalide, et tout particulièrement les extraits contenant environ 7 % de ginkgolides A, B, C et J, 50 % de flavoneglycosides et 6 % de bilobalide.

L'invention concerne également l'utilisation d'un composé de formule générale **(I)** 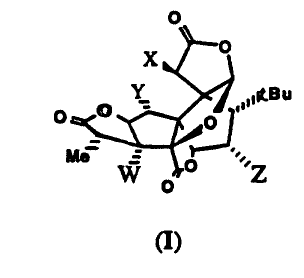 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S},
pour préparer un médicament destiné à traiter la SLA.

L'invention concerne de préférence l'utilisation d'un composé de formule générale (**I**) 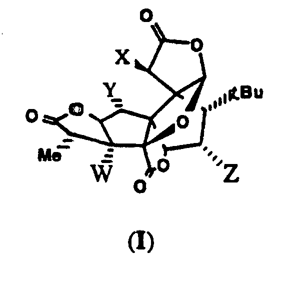 dans laquelle X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S},
pour préparer un médicament destiné à traiter la SLA.

L'invention concerne tout particulièrement l'utilisation d'un composé de formule générale (**I**) 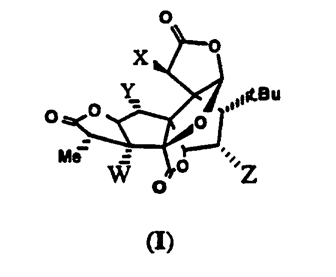 dans laquelle X représente un radical OH ou O-G_{S}, G_{S}-OH représentant un mono- ou un disaccharide, ou un de leurs dérivés ou analogues, et :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S},
pour préparer un médicament destiné à traiter la SLA.

Par radical alkoxy linéaire ou ramifié, on entend dans la présente description un radical alkoxy dont la chaine carbonée, linéaire ou ramifiée, compte de 1 à 6 atomes de carbone. Par dérivé ou analogue des mono- ou disaccharides, on entend des composés comme la N-acétylglucosamine, la N-acétylalosamine, la galactosamine, la mannoseamine, la N-tosylhydrazone, etc.

De préférence, O-G_{S} sera choisi de telle sorte que G_{S}-OH appartienne au groupe composé de l'abéquose, du rhamnose, de l'arabinose, du ribose, du xylose, du 2-déoxy-ribose, du glucose, du galactose, du mannose, du 2-déoxyglucose, du fructose, du fucose, de la N-acétylglucosamine, de la N-acétylalosamine, de la galactosamine, de la mannosamine, du saccharose, du lactose, du maltose, du cellobiose et du tréhalose. De façon encore plus préférentielle, O-G_{S} sera choisi de telle sorte que G_{S}-OH appartienne au groupe composé du glucose et du lactose.

Les différents procédés pour obtenir les dérivés glycosylés des ginkgolides ou des ginkgolides alkoxylés (c'est à dire ceux résultant d'une réaction de glycosylation effectuée sur au moins un des groupes OH des ginkgolides ou de leurs dérivés alkoxylés) sont décrits dans la publication suivante : Weber, M. et Vasella, A., *Helv. Chim. Acta*, **80** (1997), 2352-2367.

Selon une variante de l'invention, on peut également associer l'extrait de ginkgo à l'un au moins des composés suivants : le riluzole, la gabapentine, et la 1-(2-napht-2-yléthyl)-4-(3-trifluorométhylphényl)-1,2,3,6-tétrahydropyridine (aussi connue sous le nom de code SR-57746 A) ou ses sels pharmaceutiquement acceptables. L'invention concerne donc un produit comprenant au moins un extrait de ginkgo en association avec au moins un composé choisi parmi le groupe composé du riluzole, de la gabapentine, de la 1-(2-napht-2-yléthyl)-4-(3-trifluorométhylphényl)-1,2,3,6-tétrahydropyridine et de ses sels pharmaceutiquement acceptables, pour une utilisation thérapeutique simultanée, séparée ou étalée dans le temps, dans le traitement de la SLA. De préférence, on associera au moins un extrait de ginkgo avec du riluzole ou au moins un extrait de ginkgo avec de la 1-(2-napht-2-yléthyl)-4-(3-trifluorométhylphényl)-1,2,3,6-tétrahydropyridine. De façon particulièrement préférée, on associera au moins un extrait de ginkgo avec du riluzole et de la 1-(2-napht-2-yléthyl)-4-(3-trifluorométhylphényl)-1,2,3,6-tétrahydropyridine.

Optionnellement, tous les produits précédemment cités peuvent également comprendre de la vitamine E.

Par utilisation thérapeutique simultanée, on entend dans la présente demande une administration de plusieurs principes actifs par la même voie et au même moment. Par utilisation séparée, on entend notamment une administration de plusieurs principes actifs sensiblement au même moment par des voies différentes. Par utilisation thérapeutique étalée dans le temps, on entend une administration de plusieurs principes actifs à des moments différents et notamment un mode d'administration selon lequel l'ensemble de l'administration de l'un des principes actifs est effectué avant que l'administration de l'autre ou des autres ne commence. On peut ainsi administrer l'un des principes actifs pendant plusieurs mois avant d'administrer l'autre ou les autres principes actifs. Il n'y a pas de traitement simultané dans ce cas.

Les compositions pharmaceutiques comprenant un composé de l'invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques comprenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection (intramusculaire, sous-cutanée, intraveineuse,etc.), etc.

La dose d'administration journalière envisagée pour les principes actifs utilisables selon l'invention est comprise entre 0,1 mg à 10 g suivant le type de principe actif et la gravité de la SLA du sujet à traiter. De préférence, pour les extraits de Ginkgo biloba, on optera pour une dose comprise entre 20 et 200 mg par jour environ. Pour le riluzole, la dose préférée sera de l'ordre de 50 à 150 mg par jour environ, et pour la gabapentine, de l'ordre de 500 à 3 000 mg par jour environ. En ce qui concerne la 1-(2-napht-2-yléthyl)-4-(3-trifluorométhylphényl)-1,2,3,6-tétrahydropyridine, la dose préférée sera comprise entre 1 à 5 mg par jour environ, et de préférence comprise entre 1 et 3 mg par jour. Enfin, la vitamine E sera de préférence administrée à une dose de 100 à 500 mg par jour environ.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Afin de montrer l'intérêt de l'utilisation d'extraits de ginkgo dans le traitement de la SLA, les tests suivants ont été menés :

### Partie pharmacologique

### Description des tests

Pour le test qui suit, les souris sont partagées en deux groupes de souris mâles génétiquement modifiées :
- le premier groupe témoin reçoit uniquement une alimentation normale ;
- le second reçoit avec son alimentation une dose de 50 mg/kg/jour d'EGb 761 (correspondant à une alimentation comportant 0,022 % d'EGb 761).

### 1) Survie et performances motrices :

Pour ce test, on observe le comportement moteur des animaux. Tous les jours, on procède au test suivant : on place les souris sur le côté, et on sacrifie celles qui ne sont pas capables de se relever en 10 secondes. Leur durée de vie est alors comparée à la durée de vie du groupe témoin de souris mâles génétiquement modifiées recevant une alimentation normale.

On obtient les résultats suivants :
- groupe témoin : durée de vie moyenne 126 jours ;
- groupe recevant une alimentation comportant de l'EGb 761 : durée de vie 137,9 jours.

### 2) Test du "rotarod" :

Pour ce test, les souris utilisées sont partagées en trois groupes:
- le premier groupe de souris mâles génétiquement modifiées reçoit avec son alimentation une dose de 50 mg/kg d'EGb 761 (correspondant à une alimentation comportant 0,022 % d'EGb 761) ;
- le second groupe de souris mâles génétiquement modifiées reçoit uniquement une alimentation normale ;
- le troisième groupe de souris mâles non génétiquement modifiées reçoit une alimentation normale..

On place les souris dans une roue aux barreaux de laquelle elles peuvent s'accrocher. On fait tourner la roue à une vitesse de 1 tour/min, puis toutes les 10 secondes on augmente à chaque fois cette vitesse de 1 tour/min. jusqu'à ce que la souris tombe. Après trois essais, on note le temps pendant lequel la souris reste accrochée à la roue. Les souris sont testées à partir du jour 100.

Afin d'obtenir des résultats reproductibles, on laisse deux jours aux souris pour comprendre le processus avant de noter les résultats. Le test est arrêté lorsque les souris n'arrivent plus à se tenir même à une vitesse de rotation de 1 tour/min.

On obtient les résultats suivants, exprimés en secondes :

| **Jours** | **1er groupe** | **2ème groupe** | **3ème groupe** |
|---|---|---|---|
| 101 | 180,00 | 180,00 | 180,00 |
| 104 | 162,33 | 180,00 | 180,00 |
| 108 | 147,83 | 129,00 | 180,00 |
| 111 | 128,00 | 73,00 | 180,00 |
| 115 | 88,00 | 21,00 | 180,00 |
| 118 | 75,33 | 8,60 | 180,00 |
| 122 | 36,33 | 4,67 | 180,00 |

### Souris utilisées

Pour les deux tests, on utilise des souris mâles génétiquement modifiées G93A telles que décrites notamment dans les publications suivantes : Gurney M.E. et coll., *Ann. Neurol.,* **39** (1996), 147-157 ; et Gurney M.E. et coll., *Science*, **264** (1994), 1772-1775.
Les souris utilisées sont âgées de 50 à 60 jours au début du premier test, et de 100 jours au début du second.

Conclusion : les tests indiqués ci-dessus prouvent la très bonne activité de l'extrait de ginkgo dans le traitement de l'ALS.

## Revendications

1. Utilisation d'un extrait de Ginkgo biloba pour préparer un médicament destiné à traiter la sclérose latérale amyotrophique.

2. Utilisation selon la revendication 1, **caractérisé en ce que** l'extrait de Ginkgo biloba est un extrait de type EGb 761.

3. Utilisation selon la revendication 1, **caractérisé en ce que** l'extrait de Ginkgo biloba est un extrait de type CP 401.

4. Utilisation d'un composé de formule générale (**I**) 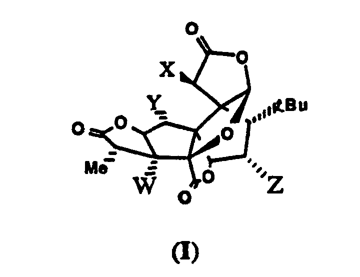 dans laquelle W, X, Y et Z représentent indépendamment les radicaux H, OH, alkoxy linéaire ou ramifié ou O-G_{S}, G_{S}-OH représentant un mono- ou un disaccharide, ou un de leurs dérivés ou analogues,
étant entendu que l'un au moins de W, X, Y ou Z représente un radical O-G_{S},
pour préparer un médicament destiné à traiter la sclérose latérale amyotrophique.

5. Utilisation selon la revendication 4, **caractérisée en ce que** :
- ou bien W représente un radical OH ou O-G_{S}, Y représente H et Z représente H ;
- ou bien W représente un radical OH ou O-G_{S}, Y représente un radical OH ou O-G_{S} et Z représente H ;
- ou bien W représente un radical OH ou O-G_{S}, Y représente un radical OH ou O-G_{S} et Z représente un radical OH ou O-G_{S} ;
- ou bien W représente un radical OH ou O-G_{S}, Y représente H et Z représente un radical OH ou O-G_{S} ;
- ou bien W représente H, Y représente un radical OH ou O-G_{S} et Z représente un radical OH ou O-G_{S} ;
- ou bien W représente un radical OH ou O-G_{S}, Y représente un radical alkoxy linéaire ou ramifié et Z représente H ;
étant entendu que l'un au moins de W, X, Y ou Z représente un radical O-G_{S}.

6. Produit comprenant au moins un extrait de ginkgo en association avec au moins un composé choisi parmi le groupe composé du riluzole, de la gabapentine, de la 1-(2-napht-2-yléthyl)-4-(3-trifluorométhylphényl)-1,2,3,6-tétrahydropyridine et de ses sels pharmaceutiquement acceptables, pour une utilisation thérapeutique simultanée, séparée ou étalée dans le temps, dans le traitement de la sclérose latérale amyotrophique.

7. Produit selon la revendication 6, **caractérisé en ce qu'**il comprend au moins un extrait de ginkgo en association avec du riluzole.

8. Produit selon la revendication 6, **caractérisé en ce qu'**il comprend au moins un extrait de ginkgo en association avec de la 1-(2-napht-2-yléthyl)-4-(3-trifluorométhylphényl)-1,2,3,6-tétrahydropyridine.

9. Produit selon l'une des revendications 6 à 8, **caractérisé en ce qu'**il comprend au moins un extrait de ginkgo en association avec du riluzole et de la 1-(2-napht-2-yléthyl)-4-(3-trifluorométhylphényl)-1,2,3,6-tétrahydropyridine.

10. Produit selon l'une des revendications 6 à 9, **caractérisé en ce qu'**il comprend en outre de la vitamine E.

## Patentansprüche

1. Verwendung eines Extrakts von Ginkgo biloba für die Herstellung eines Medikaments für die Behandlung der amyotrophen Lateralsklerose.

2. Verwendung nach Anspruch 1, **dadurch gekennzeichnet, daß** der Ginkgo-biloba-Extrakt ein Extrakt vom Typ EGb 761 ist.

3. Verwendung nach Anspruch 1, **dadurch gekennzeichnet, daß** der Ginkgo-biloba-Extrakt ein Extrakt vom Typ CP 401 ist.

4. Verwendung einer Verbindung der allgemeinen Formel (I) 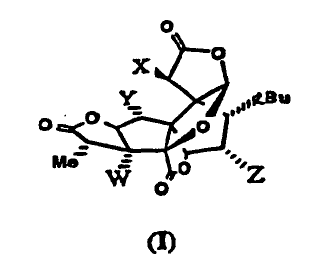 in der bedeuten W, X, Y und Z unabhängig voneinander die Reste H, OH, lineares oder verzweigtes Alkoxy oder O-G_{S}, wobei G_{S}-OH ein Mono- oder ein Disaccharid darstellt, oder eines ihrer Derivate oder Analoge,
wobei gilt, daß mindestens einer der Reste W, X, Y oder Z einen Rest O-G_{S} darstellt,
zur Herstellung eines Medikaments für die Behandlung der amyotrophen Lateralsklerose.

5. Verwendung nach Anspruch 4, **dadurch gekennzeichnet, daß**
- entweder W einen Rest OH oder O-G_{S} darstellt, Y H darstellt und Z H darstellt;
- oder W einen Rest OH oder O-G_{S} darstellt, Y einen Rest OH oder O-G_{S} darstellt und Z H darstellt;
- oder W einen Rest OH oder O-G_{S} darstellt, Y einen Rest OH oder O-G_{S} darstellt und Z einen Rest OH oder O-G_{S} darstellt;
- oder W einen Rest OH oder O-G_{S} darstellt, Y H darstellt und Z einen Rest OH oder O-G_{S} darstellt;
- oder W H darstellt, Y einen Rest OH oder O-G_{S} darstellt und Z einen Rest OH oder O-G_{S} darstellt;
- oder W einen Rest OH oder O-G_{S} darstellt, Y einen linearen oder verzweigten Alkoxyrest darstellt und Z H darstellt;
wobei gilt, daß mindestens einer der Reste W, X, Y oder Z einen Rest O-G_{S} darstellt.

6. Produkt, das mindestens einen Ginkgo-biloba-Extrakt in Kombination mit mindestens einer Verbindung enthält, die aus der Gruppe ausgewählt ist, die aus Riluzol, Gabapentin, 1-(2-Napht-2-ylethyl)-4-(3-trifluormethylphenyl)-1,2,3,6-tetrahydropyridin und deren pharmazeutisch verträglichen Salzen besteht, für eine gleichzeitige, getrennte oder zeitlich verschobene therapeutische Verwendung bei der Behandlung der amyotrophen Lateralsklerose.

7. Produkt nach Anspruch 6, **dadurch gekennzeichnet, daß** es mindestens einen Ginkgo-Extrakt in Kombination mit Riluzol enthält.

8. Produkt nach Anspruch 6, **dadurch gekennzeichnet, daß** es mindestens einen Ginkgo-Extrakt in Kombination mit 1-(2-Napht-2-ylethyl)-4-(3-trifluormethylphenyl)-1,2,3,6-tetrahydropyridin enthält.

9. Produkt nach einem der Ansprüche 6 bis 8, **dadurch gekennzeichnet, daß** es mindestens einen Ginkgo-Extrakt in Kombination mit Riluzol und 1-(2-Napht-2-ylethyl)-4-(3-trifluormethylphenyl)-1,2,3,6-tetrahydropyridin enthält.

10. Produkt nach einem der Ansprüche 6 bis 9, **dadurch gekennzeichnet, daß** es außerdem Vitamin E enthält.

## Claims

1. Use of an extract of Ginkgo biloba for preparing a medicament intended to treat amyotrophic lateral sclerosis.

2. Use according to claim 1, **characterized in that** the extract of Ginkgo biloba is an extract ofEGb 761 type.

3. Use according to claim 1, **characterized in that** the extract of Ginkgo biloba is an extract of CP 401 type.

4. Use of a compound of general formula (**I**) 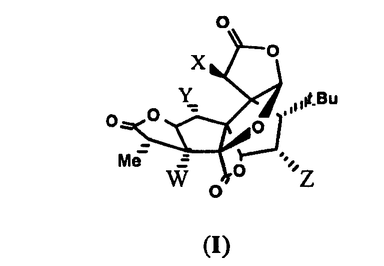 in which W, X, Y and Z represent independently the H, OH, linear or branched alkoxy or O-G_{S} radicals, G_{S}-OH representing a mono- or a disaccharide, or one of their derivatives or analogues,
it being understood that at least one of W, X, Y or Z represents an O-G_{S} radical,
for preparing a medicament intended to treat amyotrophic lateral sclerosis.

5. Use according to claim 4, **characterized in that**:
- either W represents an OH or O-G_{S} radical, Y represents H and Z represents H;
- or W represents an OH or O-G_{S} radical, Y represents an OH or O-G_{S} radical and Z represents H;
- or W represents an OH or O-G_{S} radical, Y represents an OH or O-G_{S} radical and Z represents an OH or O-G_{S} radical;
- or W represents an OH or O-G_{S} radical, Y represents H and Z represents an OH or O-G_{S} radical;
- or W represents H, Y represents an OH or O-G_{S} radical and Z represents an OH or O-G_{S} radical;
- or W represents an OH or O-G_{S} radical, Y represents a linear or branched alkoxy radical and Z represents H;
it being understood that at least one of W, X, Y or Z represents an O-G_{S} radical.

6. Product comprising at least one extract of ginkgo in combination with at least one compound chosen from the group composed of riluzole, gabapentin, 1-(2-naphth-2-ylethyl)-4-(3-trifluoromethylphenyl)-1,2,3,6-tetrahydropyridine or its pharmaceutically acceptable salts, for a therapeutic use which is simultaneous, separated or spread out over time, in the treatment of amyotrophic lateral sclerosis.

7. Product according to claim 6, **characterized in that** it comprises at least one extract of ginkgo in combination with riluzole.

8. Product according to claim 6, **characterized in that** it comprises at least one extract of ginkgo in combination with 1-(2-naphth-2-ylethyl)-4-(3-trifluoromethylphenyl)-1,2,3,6-tetrahydropyridine.

9. Product according to one of claims 6 to 8, **characterized in that** it comprises at least one extract of ginkgo in combination with riluzole and 1-(2-naphth-2-ylethyl)-4-(3-trifluoromethylphenyl)-1,2,3,6-tetrahydropyridine.

10. Product according to one of claims 6 to 9, **characterized in that** it comprises in addition vitamin E.
